# FASCICULE DE BREVET EUROPEEN

(11) **EP 1 027 879 B1**
(45) Date de publication et mention de la délivrance du brevet: **20.04.2005**
(21) Numéro de dépôt: 00400084.0
(22) Date de dépôt: 13.01.2000
(51) Int. Cl.: A61K 7/13, A61K 7/02

(54) **Utilisation de composés polycycliques condensés cationiques en teinture des matières kératiniques, compositions tinctoriales et procédé de teinture**
Verwendung kationischer kondensierter polyzyklischer Verbindungen zur Färbung keratinischer Materialien, Färbezusammensetzungen und Verfahren
Use of cationic condensed polycyclic compounds for dyeing of keratinic substances, dyeing compositions and process

(30) Priorité: 09.02.1999 FR 9901504; 02.04.1999 FR 9904185
(43) Date de publication de la demande: 16.08.2000
(73) Titulaire: L'OREAL, 75008 Paris (FR)
(72) Inventeur: Vandenbossche, Jean-Jacques, 40400 Tartas (FR); Lagrange, Alain, 77700 Coupvray (FR)
(74) Mandataire: Le Blainvaux Bellegarde, Françoise

(56) Documents cités:
- DE-A- 2 147 706
- FR-A- 1 557 945
- US-A- 4 139 532

## Description

L'invention a pour objet l'utilisation de composés polycycliques condensés comportant au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé, à titre de colorants directs dans des compositions destinées à la teinture des matières kératiniques et en particulier des compositions destinées à la teinture des fibres kératiniques humaines et notamment des cheveux, et des compositions cosmétiques destinées au maquillage de la peau, des ongles et des lèvres, les compositions de teinture ou de maquillage les contenant et le procédé de teinture directe correspondant.

Dans le domaine de la teinture capillaire, on recherche des colorants directs, c'est-à-dire des colorants qui, sans l'apport d'agent oxydant, sont capables de modifier par eux-mêmes la nuance naturelle des cheveux, de façon temporaire. Dans cette application, les colorants doivent satisfaire à un certain nombre de critères, et notamment engendrer des teintures reproductibles avec des nuances riches et variées permettant d'obtenir une large palette de couleurs susceptible de satisfaire le formulateur, ces teintures devant en outre être puissantes et résistantes au lavage, au frottement, à l'ondulation permanente, à la lumière et à la transpiration.

Or, la demanderesse vient maintenant de découvrir, de façon totalement inattendue et surprenante, que des composés polycycliques condensés de formule (I) ci-après définie, comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, conviennent pour une utilisation comme colorant direct pour la teinture directe, mais en outre qu'ils permettent d'obtenir des compositions tinctoriales conduisant à des colorations puissantes et variées présentant d'excellentes propriétés de résistance aux différents traitements que peuvent subir les fibres kératiniques, et notamment vis à vis de la lumière, des lavages, de l'ondulation permanente et de la transpiration. Enfin, ces composés présentent une meilleure solubilité dans les milieux classiquement utilisés pour la teinture des fibres kératiniques et s'avèrent être aisément synthétisables.

Ces découvertes sont à la base de la présente invention.

L'invention a donc pour premier objet, l'utilisation, à titre de colorants directs dans des, ou pour la fabrication de, compositions tinctoriales pour matières kératiniques, et notamment pour fibres kératiniques humaines telles que les cheveux, de composés polycycliques condensés de formule (I) suivante, et leurs sels d'addition avec un acide : 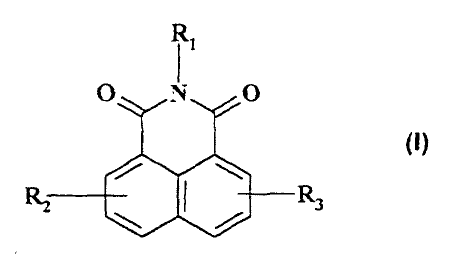 dans laquelle :
- R₁, représente un atome d'hydrogène ; un groupement Z défini ci-après ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆ ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) : un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ;
- R₂ et R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical hydroxyle ; un radical nitro ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical amino ; un radical N-alkyl(C₁-C₆)amino ; un radical N,N-dialkyl(C₁-C₆)amino (les deux substituants alkyle pouvant former un cycle à 5 ou 6 chaînons) ; un radical N-. hydroxyalkyl(C₁-C₆)amino ; un radical N,N-bis(hydroxyalkyl(C₁-C₆))amino ; un radical N-polyhydroxyalkyl(C₂-C₆)amino ; un radical N,N-bis(polyhydroxyalkyl (C₂-C₆))amino ; un radical aminoalkyl(C₁-C₆)amino dans lequel le groupement amino terminal est non substitué ou substitué par un ou deux radicaux alkyle en C₁-C₆, lesdits radicaux alkyle pouvant former un cycle saturé ou non à 5 ou 6 chaînons ; un groupe amino protégé par un radical alkyl(C₁-C₆)carbonyle, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, formyle ; un groupement OR₄ ou SR₄ ;
- R₄ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, alkyl(C₁-C₆)sulfonyle.
- Les substituants R₂ et R₃ peuvent former ensemble un cycle insaturé de structure (II) suivante : 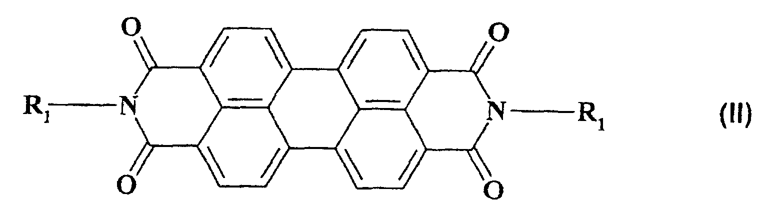
- Z est choisi parmi les groupements cationiques insaturés de formules (III) et (IV) suivantes, et les groupements cationiques saturés de formule (V) suivante : 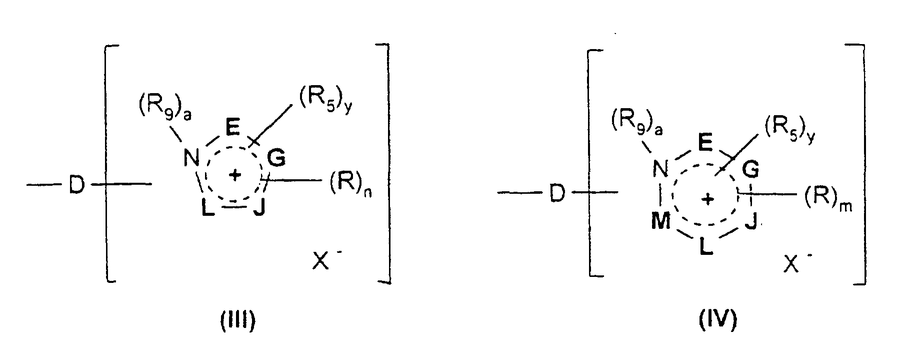 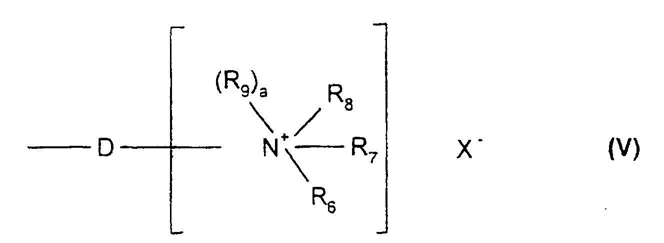 dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent une liaison covalente, un groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR''R''' dans lesquels R" et R"', identiques ou différents, représentent un radical alkyle en C₁-C₆, un radical monohydroxyalkyle en C₁-C₆ ou un radical polyhydroxyalkyle en C₂-C₆ ; lorsque dans les formules (III) ou (IV), n ou m est supérieur à 2, deux radicaux R adjacents peuvent également former ensemble un cycle insaturé à 5 ou 6 chaînons, carboné, ou contenant un ou plusieurs hétéroatomes; parmi lesdits hétérocycles, on peut citer à titre d'exemple, les groupements pyrazoltriazolinium, pyrazoloimidazolinium, pyrrolotriazolinium, pyrazolopyrimidinium, pyrazolopyridinium, benzoimidazolinium, benzoxazolinium, benzothiazolinium, indolinium, indolidinium, isoindolinium, indazolinium, benzotriazolinium, quinolinium, tétrahydroquinolinium, benzoimidazolidinium, benzopyridinium ;
   - R₅ représente une liaison covalente, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un groupement Z ;
   - R₆, R₇ et R₈, identiques ou différents, représentent une liaison covalente, un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₆, R₇ et R₈ peuvent également former ensemble, avec l'atome d'azote auquel ils sont rattachés, un cycle saturé à 5 ou 6 chaînons, carboné, ou contenant un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
      l'un des radicaux R₆, R₇ et R₈ peut également représenter un second groupement Z, identique ou différent du premier groupement Z ;
   - R₉ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
   - a et y sont des nombres entiers égaux à 0 ou 1 ; avec les conditions suivantes :
      - dans les groupements cationiques insaturés de formule (III):
         - lorsque a = 0, le bras de liaison D est rattaché à l'atome d'azote,
         - lorsque a = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₅ est porté par l'atome d'azote du cycle insaturé ; ou bien
            2) lorsqu'au moins un des sommets E, G, J et L représente un atome d'azote sur lequel le radical R₅ est fixé ;
      - dans les groupements cationiques insaturés de formule (IV) :
         - lorsque a = 0, le bras de liaison D est rattaché à l'atome d'azote,
         - lorsque a = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₅ est porté par l'atome d'azote du cycle insaturé ;
      - dans les groupements cationiques de formule (V) :
         - lorsque a = 0, alors le bras de liaison D est rattaché à l'atome d'azote portant les radicaux R₇ à R₈,
         - lorsque a = 1, alors deux des radicaux R₇ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
   - X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu que :**
- le nombre de groupements cationiques Z de formule (III), (IV) ou (V) est au moins égal à 1.

Dans les formules (I), (II), (III), (IV) et (V) ci-dessus les radicaux alkyle et alcoxy peuvent être linéaires ou ramifiés.
Les composés de formule (I) peuvent être éventuellement salifiés par des acides minéraux forts tels que HCl, HBr, H₂SO₄, ou des acides organiques tels qu'acétique, tartrique, lactique, citrique ou succinique.

Parmi les cycles des groupements insaturés Z de formule (III) ci-dessus, on peut notamment citer à titre d'exemple les cycles pyrrolique, imidazolique, pyrazolique, oxazolique, thiazolique et triazolique.

Parmi les cycles des groupements insaturés Z de formule (IV) ci-dessus, on peut notamment citer à titre d'exemple les cycles pyridinique, pyrimidinique, pyrazinique, oxazinique et triazinique.

Parmi les composés de formule (I) et (II) ci-dessus, on peut notamment citer les composés suivants :
Anthra[2,1,9-def:6,5,10-d'e'f']diisoquinoline-2,9-dipropanaminium, N,N'-dihexyl-1,3,8,10-tetrahydro-N,N,N',N'-tetraméthyl-1,3,8,10-tétraoxo, diiodure ;
Morpholinium, 4,4'-[(1,3,8,10-tétrahydro-1,3,8,10-tétraoxoanthra[2,1,9-def:6,5,10-d'e'f]diisoquinoline-2,9-diyl)di-3,1-propanediyl]bis[4-méthyl], diiodure ;
Anthra[2,1,9-def:6,5,10-d'e'f']diisoquinoline-2,9-dipropanaminium, N,N,N',N'-tétraéthyl-1,3,8,10-tétrahydro-N,N'-diméthyl-1,3,8,10-tétraoxo, diiodure ;
Anthra[2,1,9-def:6,5,10-d'e'f']diisoquinoline-2,9-diéthanaminium, N,N,N,N',N',N'-hexaéthyl-1,3,8,10-tétrahydro-1,3,8,10-tétraoxo, diiodure ;
Anthra[2,1,9-def:6,5,10-d'e'f']diisoquinoline-2,9-dipropanaminium, N,N'-diéthyl-1,3,8,10-tétrahydro-N,N,N',N'-tétraméthyl-1,3,8,10-tétraoxo, diiodure ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, diiodure ;
Anthra[2,1,9-def:6,5,10-d'e'f']diisoquinoline-2,9-dipropanaminium, 1,3,8,10-tétrahydro-N,N,N,N',N',N'-hexaméthyl-1,3,8,10-tétraoxo, diiodure ;
Anthra[2,1,9-def:6,5,10-d'e'f']diisoquinoline-2,9-diéthanaminium, N,N,N,N',N',N'-hexaéthyl-1,3,8,10-tétrahydro-1,3,8,10-tétraoxo, dibenzenesulfonate ;
Anthra[2,1,9-def:6,5,10-d'e'f']diisoquinoline-2,9-diéthanaminium, 1,3,8,10-tétrahydro-N,N,N,N',N',N'-hexaéthyl-1,3,8,10-tétraoxo, diiodure ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, dibenzenesulfonate ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diméthosulfate.

Parmi ces composés de formule (I) ou (II), on préfère plus particulièrement :
Anthra[2,1,9-def:6,5,10-d'e'f']diisoquinoline-2,9-dipropanaminium, 1,3,8,10-tétrahydro-N,N,N,N',N',N'-hexaméthyl-1,3,8,10-tetraoxo, diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diméthosulfate.

Les composés de formule (I) ou (II) conformes à l'invention peuvent être facilement obtenus selon des méthodes bien connues de l'état de la technique pour l'obtention des amines quaternisées, par exemple :
- en un temps, par condensation d'un composé comportant un radical halogénoalkyle avec un composé porteur d'un radical amine tertiaire, ou par condensation d'un composé comportant un radical amine tertiaire avec un composé porteur d'un radical halogénoalkyle ;
- ou en deux temps, par condensation d'un composé comportant un radical halogénoalkyle avec un composé porteur d'une amine secondaire, ou par condensation d'un anhydride avec une amino(di-substituée)alkyleamine (schéma ci-dessous), suivie d'une quaternisation avec un agent alkylant.

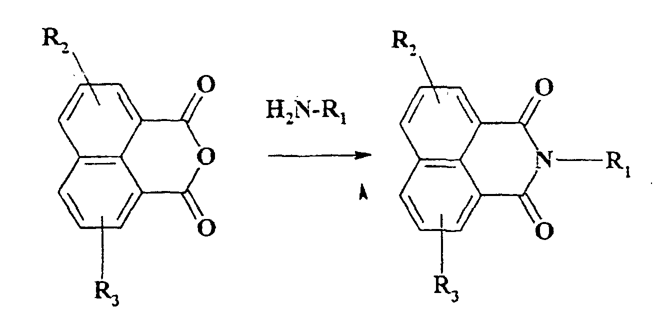

L'étape de quaternisation est, généralement par commodité, la dernière étape de la synthèse, mais peut intervenir plus tôt dans la suite des réactions conduisant à la préparation des composés de formule (I) ou (II).

L'invention a également pour objet une composition de teinture ou de maquillage des matières kératiniques, caractérisée par le fait qu'elle comprend, dans un milieu approprié pour la teinture ou le maquillage, une quantité efficace d'au moins un composé de formule (I) conforme à l'invention.

Au sens de la présente invention, on entend principalement par matières kératiniques, la peau du visage ou du corps, les lèvres, les ongles, les fibres kératiniques humaines telles que les cheveux humains, les poils, cils, sourcils, et également les fibres kératiniques telles que les fibres textiles naturelles dont notamment la laine.

L'invention a en particulier pour objet une composition pour la teinture directe des fibres kératiniques humaines telles que les cheveux, caractérisée par le fait qu'elle comprend, dans un milieu approprié pour la teinture, une quantité efficace d'au moins un composé de formule (I) conforme à l'invention.

Lorsque la composition est destinée à la teinture, le ou les composés polycycliques condensés cationiques de formule (I) conformes à l'invention et/ou le ou leurs sels d'addition avec un acide représentent de préférence de 0,005 à 12 % en poids environ du poids total de la composition tinctoriale, et encore plus préférentiellement de 0,05 à 6 % en poids environ de ce poids.
Lorsque la composition est destinée au maquillage, le ou les composés polycycliques condensés cationiques de formule (I) conformes à l'invention et/ou le ou leurs sels d'addition avec un acide représentent de préférence de 0,005 à 25 % en poids environ du poids total de la composition, et encore plus préférentiellement de 0,05 à 15 % en poids environ de ce poids.

Les composés de formule (I) conformes à l'invention peuvent également servir, dans les procédés bien connus de teinture d'oxydation des fibres kératiniques humaines, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les composés polycycliques condensés cationiques de formule (I) selon l'invention, d'autres colorants directs classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, des colorants mono- ou di-azoïques, triarylméthaniques, aziniques, acridiniques et xanthéniques, ou encore des colorants métallifères.

La proportion de tous ces autres colorants directs d'addition peut varier entre 0,5 et 10% en poids environ par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Il peut aussi contenir des corps gras tels que des huiles et des cires.

Les solvants peuvent être présents dans des proportions de préférence comprises entre 1 et 40 % en poids environ par rapport au poids total de la composition tinctoriale, et encore plus préférentiellement entre 5 et 30 % en poids environ.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le pH de la composition tinctoriale conforme à l'invention destinée à la teinture . des fibres kératiniques est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 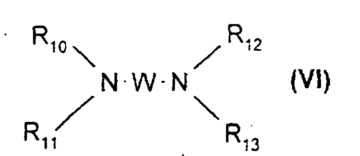 dans laquelle W est un reste propylène éventuellement substitué par un groupement hydroxyle ou un radical alkyle en C₁-C₆ ; R₁₀, R₁₁, R₁₂ et R₁₃, identiques ou différents, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquides, de crèmes, de gels, de cataplasmes, ou sous toute autre forme appropriée pour réaliser une teinture des matières kératiniques, et notamment des cheveux humains. En particulier, elle peut être conditionnée sous pression en flacon aérosol en présence d'un agent propulseur et former une mousse.

Les compositions cosmétiques destinées au maquillage sont notamment des produits de maquillage pour le visage ou les lèvres tels que les fards à paupière ou à joue, les poudres et blushes, les fonds de teint, les bâtons ou laques à lèvres et les produits de maquillage du corps humain; elles sont aussi des produits de maquillage des cils, des sourcils et des ongles, tels que les mascaras, les crayons pour sourcils, les ligneurs encore appelés "eye-liner" et les vernis à ongles.

Un autre objet de l'invention porte sur un procédé de teinture des fibres kératiniques et en particulier des fibres kératiniques humaines telles que les cheveux, par coloration directe, consistant à laisser agir une composition renfermant au moins un composé polycyclique condensé cationique de formule (I) sur les fibres kératiniques sèches ou humides.
On peut utiliser la composition selon l'invention en tant que composition non rincée, c'est-à-dire qu'après application de la composition sur les fibres, on sèche sans rinçage intermédiaire.
Dans les autres modes d'application, après application de la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLES DE COMPOSITIONS TINCTORIALES

### EXEMPLES 1 à 2 :

On a préparé les 2 compositions de teinture directe pour cheveux réunies dans le tableau suivant *(toutes teneurs exprimées en grammes - M.A. désigne Matière Active)*:

| | **Exemple 1** | **Exemple 2** |
|---|---|---|
| Colorant de formule (I)1* | 0,890 | |
| Colorant de formule (I)2** | | 0,859 |
| Alcool benzylique | 10 | 10 |
| Alcool éthylique | 21 | 21 |
| Tampon pH 7 qs | pH 7 | pH 7 |
| Eau déminéralisée | 100 | 100 |

| | | |
|---|---|---|
| *Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1- propanediyl], diiodure ; | | |
| **Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1- propanediyl], diméthosulfate. | | |

On a appliqué chacune des compositions ci-dessus sur des mèches de cheveux gris naturels ou permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 1 | Irisé rouge |
| Composition de l'exemple 2 | Irisé rouge |

### EXEMPLES DE COMPOSITIONS DE MAQUILLAGE

### Exemple 3 : Rouge-à-lèvres :

| | |
|---|---|
| Cire de Polyéthylène | 14 g |
| Huile de Sésame | 78 g |
| Colorant de formule (I)1*^{défini dans l'exemple 1} | 5 g |
| Dioxyde de titane | 3 g |

Mode opératoire :
- Homogénéisation du mélange huile + colorant et pigment pendant 45 minutes dans un bain d'huile,
- 3 passages successifs à un broyeur tricylindre,
- Homogénéisation du mélange huile + colorant et pigment pendant 30 minutes dans un bain d'huile,
- Moulage dans moule à 42°C et 30 minutes au congélateur.

On obtient un rouge-à-lèvres d'un rouge intense.

### Exemple 4 : Blush :

| | |
|---|---|
| Talc | 38 g |
| Mica | 20 g |
| Oxychlorure de Bismuth | 8 g |
| Stéarate de zinc | 3 g |
| Poudre de nylon | 20 g |
| Colorant de formule (I)2** ^{défini dans l'exemple 2} | 5 g |
| Liant gras (*) | qsp 100g |

| | |
|---|---|
| (*) Mélange d'huiles carbonées contenant : - 3,6 g de triglycérides d'acide caprique/caprylique, - 2,0 g d'isoparaffine hydrogénée (non volatile), - 1,0 g d'huile de jojoba. | |

Mode opératoire :
- Prémélange de toutes les charges et colorant et pigments,
- 5 minutes au Lôdige (mélangeur homogénéisateur de poudres),
- Addition du liant organique,
- 5 minutes au Lödige,
- Passage au jet d'air (CHRISPRO),
- Tamisage à 160 microns.

### Exemple 5 : Fond de teint de type huile-dans-eau

### - Phase A

. Acide stéarique 2,0 %
. Stéarate de glycéryle 3,0 %
. Isostéarate de glycéryle 2,0 %
. Huile minérale 8,0 %
. Pigments
   Oxyde de fer rouge 0,9 %
   Oxyde de fer noir 0,3 %
   Dioxyde de titane 4,4 %
   Colorant de formule (I) 1*^{défini dans l'exemple 1} 0,7 %
. Conservateur 0,2 %
. Diméthicone (5cst) 4,0 %

### - Phase B

| | |
|---|---|
| Triéthanolamine | 1,0 % |

### - Phase C

. Conservateur 0,2 %
. Silicate de magnésium et d'aluminium en gel à 5% de matière active 20,0 %
. Gomme de cellulose 3,5 %
. Lauroyl sarcosinate de sodium 3,5 %
. Glycérine 2,0 %
. Eau qsp 100 %

### - Phase D

| | |
|---|---|
| Conservateur | 0,3 % |
| Eau | 2,0 % |

### - Mode opératoire :

On prépare les phase A et C séparément, on les chauffe à 80°C puis on les homogénéise à l'homogénéisateur commercialisé sous le nom de Moritz. On introduit la phase B dans la phase A, puis on verse ce mélange dans la phase C sous agitation. On ajoute ensuite la phase D et on poursuit l'agitation jusqu'à un complet refroidissement.

On obtient un fond de teint de couleur beige.

## Revendications

1. Utilisation à titre de colorants directs dans des, ou pour la fabrication de, compositions tinctoriales pour matières kératiniques, de composés polycycliques condensés de formule (I) suivante, et leurs sels d'addition avec un acide : 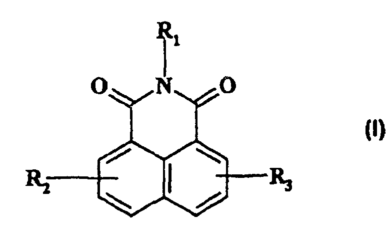 dans laquelle :
• R₁, représente un atome d'hydrogène ; un groupement Z défini ci-après ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆ ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ;
• R₂ et R₃, qui peuvent être identiques ou différents, représentent un atome d'hydrogène; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) un radical N,N-dialkyl(C₁-C₆)carbamylalkyle(C₁-C₆) ; un radical alkyle en C₁-C₆ ; un radical hydroxyle ; un radical nitro ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical amino ; un radical N-alkyl(C₁-C₆)amino ; un radical N,N-dialkyl(C₁-C₆)amino (les deux substituants alkyle pouvant former un cycle à 5 ou 6 chaînons) ; un radical N-hydroxyalkyl(C₁-C₆)amino ; un radical N,N-bis(hydroxyalkyl(C₁-C₆))amino ; un radical N-polyhydroxyalkyl(C₂-C₆)amino ; un radical N,N-bis(polyhydroxyalkyl (C₂-C₆))amino ; un radical aminoalkyl(C₁-C₆)amino dans lequel le groupement amino terminal est non substitué ou substitué par un ou deux radicaux alkyle en C₁-C₆, lesdits radicaux alkyle pouvant former un cycle saturé ou non à 5 ou 6 chaînons ; un groupe amino protégé par un radical alkyl(C₁-C₆)carbonyle, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, formyle ; un groupement OR₄ ou SR₄;
• Les substituants R₂ et R₃ peuvent former ensemble un cycle insaturé de structure (II) suivante : 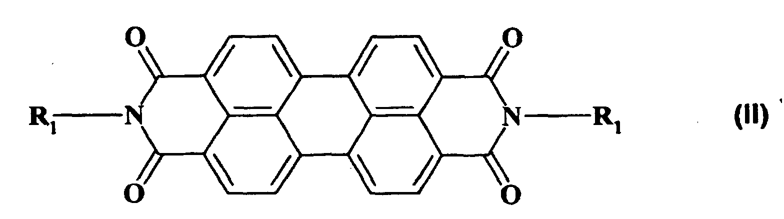
• R₄ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, alkyl(C₁-C₆)sulfonyle ;
• Z est choisi parmi les groupements cationiques insaturés de formules (III) et (IV) suivantes, et les groupements cationiques saturés de formule (V) suivante : 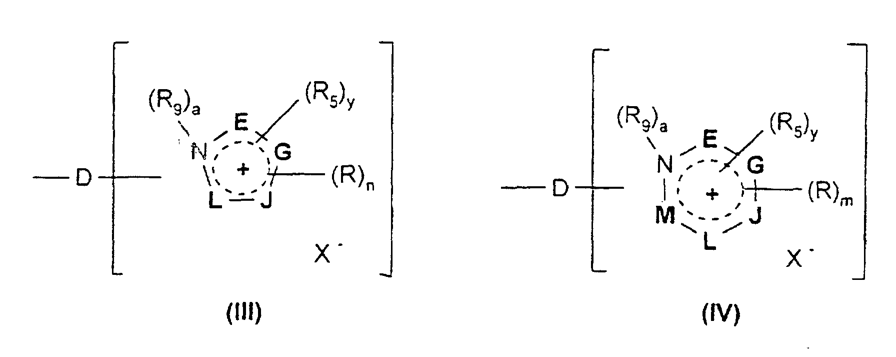 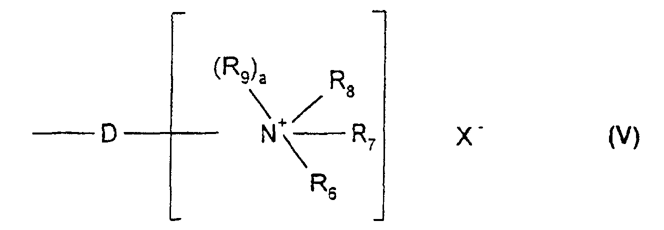 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent une liaison covalente, un groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR'' ou NR''R''' dans lesquels R" et R''', identiques ou différents, représentent un radical alkyle en C₁-C₆, un radical monohydroxyalkyle en C₁-C₆ ou un radical polyhydroxyalkyle en C₂-C₆ ; lorsque dans les formules (III) ou (IV), n ou m est supérieur à 2, deux radicaux R adjacents peuvent également former ensemble un cycle insaturé à 5 ou 6 chaînons, carboné, ou contenant un ou plusieurs hétéroatomes;
• R₅ représente une liaison covalente, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un groupement Z ;
• R₅, R₇ et R₈, identiques ou différents, représentent une liaison covalente, un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₆, R₇ et R₈ peuvent également former ensemble, avec l'atome d'azote auquel ils sont rattachés, un cycle saturé à 5 ou 6 chaînons, carboné, ou contenant un ou plusieurs hétéroatomes,
l'un des radicaux R₆, R₇ et R₈ peut également représenter un second groupement Z, identique ou différent du premier groupement Z ;
• R₉ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• a et y sont des nombres entiers égaux à 0 ou 1 ; avec les conditions suivantes :
- dans les groupements cationiques insaturés de formule (III) :
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₅ est porté par l'atome d'azote du cycle insaturé ; ou bien
2) lorsqu'au moins un des sommets E, G, J et L représente un atome d'azote sur lequel le radical R₅ est fixé ;
- dans les groupements cationiques insaturés de formule (IV) :
- lorsque a = 0, le bras de liaison D est rattaché à l'atome d'azote,
- lorsque a = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₅ est porté par l'atome d'azote du cycle insaturé ;
- dans les groupements cationiques de formule (V) :
- lorsque a = 0, alors le bras de liaison D est rattaché à l'atome d'azote portant les radicaux R₇ à R₈,
- lorsque a = 1, alors deux des radicaux R₇ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X⁻ représente un anion monovalent ou divalent ;
**étant entendu que :**
- le nombre de groupements cationiques Z de formule (III), (IV) ou (V) est au moins égal à 1.

2. Utilisation selon la revendication 1, **caractérisée par le fait que** les matières kératiniques désignent la peau humaine, les lèvres et les ongles.

3. Utilisation selon la revendication 1, **caractérisée par le fait que** les matières kératiniques désignent les fibres kératiniques humaines telles que les cheveux, les poils, les cils et les sourcils.

4. Utilisation selon l'une quelconque des revendications 1 à 3, **caractérisée par le fait que** les cycles des groupements insaturés Z de formule (III) sont choisis parmi les cycles pyrrolique, imidazolique, pyrazolique, oxazolique, thiazolique et triazolique.

5. Utilisation selon l'une quelconque des revendications 1 à 3, **caractérisée par le fait que** les cycles des groupements insaturés Z de formule (IV) sont choisis parmi les cycles pyridinique, pyrimidinique, pyrazinique, oxazinique et triazinique.

6. Utilisation selon l'une quelconque des revendications précédentes, **caractérisée par le fait que** dans la formule (V) deux des radicaux R₆, R₇ et R₈ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

7. Utilisation selon l'une quelconque des revendications précédentes, **caractérisée par le fait que** X ⁻ est choisi parmi un atome d'halogène, un hydroxyde, un hydrogènesulfate, ou un alkyl(C₁-C₆)sulfate.

8. Utilisation selon l'une quelconque des revendications précédentes, **caractérisée par le fait que** dans la formule (I), R₂ et R₃ forment ensemble un cycle insaturé de formule (II) suivante : 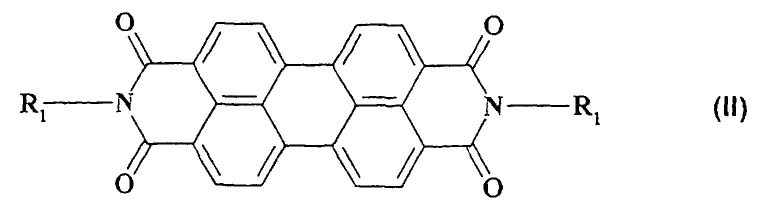 dans laquelle le radical R, a les mêmes significations que dans la formule (I).

9. Utilisation selon l'une quelconque des revendications précédentes, **caractérisée par le fait que** les composés de formule (I) sont choisis parmi les suivants :
Anthra[2,1,9-def:6,5,10-d'e'f']diisoquinoline-2,9-dipropanaminium, N,N'-dihexyl-1,3,8,10-tetrahydro-N,N,N',N'-tetraméthyl-1,3,8,10-tétraoxo, diiodure ;
Morpholinium, 4,4'-[(1,3,8,10-tétrahydro-1,3,8,10-tétraoxoanthra[2,1,9-def:6,5,10-d'e'f]diisoquinoline-2,9-diyl)di-3,1-propanediyl]bis[4-méthyl], diiodure ;
Anthra[2,1,9-def:6,5,10-d'e'f']diisoquinoline-2,9-dipropanaminium, N,N,N',N'-tétraéthyl-1,3,8,10-tétrahydro-N,N'-diméthyl-1,3,8,10-tétraoxo, diiodure ;
Anthra[2,1,9-def:6,5,10-d'e'f']diisoquinoline-2,9-diéthanaminium, N,N,N,N',N',N'-hexaéthyl-1,3,8,10-tétrahydro-1,3,8,10-tétraoxo, diiodure ;
Anthra[2,1,9-def:6,5,10-d'e'f']diisoquinoline-2,9-dipropanaminium, N,N'-diéthyl-1,3,8,10-tétrahydro-N,N,N',N'-tétraméthyl-1,3,8,10-tétraoxo, diiodure ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, diiodure ;
Anthra[2,1,9-def:6,5,10-d'e'f']diisoquinoline-2,9-dipropanaminium,1,3,8,10-tétrahydro-N,N,N,N',N',N'-hexaméthyl-1,3,8,10-tétraoxo, diiodure ;
Anthra[2,1,9-def:6,5,10-d'e'f']diisoquinoline-2,9-diéthanaminium, N,N,N,N',N',N'-hexaéthyl-1,3,8,10-tétrahydro-1,3,8,10-tétraoxo, dibenzenesulfonate ;
Anthra[2,1,9-def:6,5,10-d'e'f']diisoquinoline-2,9-diéthanaminium, 1,3,8,10-tétrahydro-N,N,N,N',N',N'-hexaéthyl-1,3,8,10-tétraoxo, diiodure ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, dibenzenesulfonate ;
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diméthosulfate.

10. Utilisation selon la revendication 9, **caractérisée par le fait que** les composés de formule (I) sont choisis parmi les suivants :
Anthra[2,1,9-def:6,5,10-d'e'f']diisoquinoline-2,9-diéthanaminium, 1,3,8,10-tétrahydro-N,N,N,N',N',N'-hexaméthyl-1,3,8,10-tétraoxo, diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diiodure ;
Imidazolinium, bis[1-1'méthyl] 1,1'-[(1,3,8,10-tétrahydro-1,3,8,10-tétraoxoanthra[2,1,9-def:6,5,10-d'e'f]diisoquinoline-2,9-diyl)di-3,1-propanediyl], diméthosulfate.

11. Utilisation selon l'une quelconque des revendications précédentes, **caractérisée par le fait que** les sels d'addition avec un acide des composés de formule (I) sont choisis parmi les chlorhydrates, les bromhydrates, les sulfates, les citrates, les succinates, les tartrates, les lactates et les acétates.

12. Composition de teinture ou de maquillage pour matières kératiniques, **caractérisée par le fait qu'**elle comprend, dans un milieu approprié pour la teinture ou le maquillage, une quantité efficace d'au moins un composé de formule (I) défini à l'une quelconque des revendications 1-11.

13. Composition de teinture directe pour fibres kératiniques humaines et notamment les cheveux, **caractérisée par le fait qu'**elle comprend, dans un milieu approprié pour la teinture, une quantité efficace d'au moins un composé de formule (I) défini à l'une quelconque des revendications 1-11.

14. Composition selon la revendication 13, **caractérisée par le fait qu'**elle a un pH compris entre 3 et 12.

15. Composition de teinture selon l'une quelconque des revendications 12 à 14, **caractérisée par le fait que** les composés de formule (I) sont présents dans une concentration allant de 0,005 à 12 % en poids par rapport au poids total de la composition.

16. Composition selon la revendication 15, **caractérisée par le fait que** les composés de formule (I) sont présents dans une concentration allant de 0,05 à 6 % en poids par rapport au poids total de la composition.

17. Composition selon l'une quelconque des revendications 13 à 16, , **caractérisée par le fait que** le milieu approprié pour la teinture est un milieu aqueux constitué par de l'eau et/ou des solvants organiques, dans des proportions comprises entre 1 et 40% en poids par rapport au poids total de la composition.

18. Composition de maquillage selon la revendication 12, **caractérisée par le fait que** les composés de formule (I) sont présents dans une concentration allant de 0,005 à 25 % en poids par rapport au poids total de la composition.

19. Composition selon la revendication 12 ou 18, **caractérisée par le fait qu'**elle se présente sous la forme d'un produit de maquillage de la peau humaine, des lèvres, des ongles, ou des cils et des sourcils.

20. Composition selon la revendication 19, **caractérisée par le fait qu'**elle se présente sous la forme d'un fond de teint, d'un fard à joue, ou à paupières, d'une poudre ou blush, d'un rouge à lèvres ou brillant à lèvres, d'un vernis à ongles, d'un mascara, d'un eye-liner.

21. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 13 à 17, sur les fibres kératiniques sèches ou humides, et qu'on sèche ces fibres sans rinçage intermédiaire.

22. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 13 à 17 sur les fibres kératiniques sèches ou humides, et qu'après avoir éventuellement laissé agir la composition sur les fibres pendant un temps de pose allant de 3 à 60 minutes, on rince les fibres, on les lave éventuellement, on les rince à nouveau puis on les sèche.

## Patentansprüche

1. Verwendung von polycyclischen kondensierten Verbindungen der folgenden Formel (I) und deren Additionssalzen mit einer Säure in Zusammensetzungen zum Färben von Keratinsubstanzen oder zu deren Herstellung: 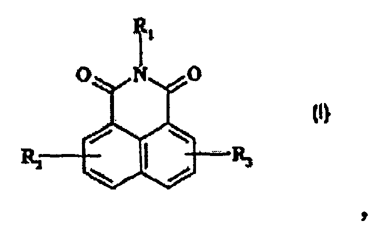 worin bedeuten:
• R₁ ein Wasserstoffatom; eine nachstehend definierte Gruppe Z: Aminoalkyl(C₁₋₆)carbonylalkyl(C₁₋₆); N-Z-Aminoalkyl(C₁₋₆)carbonylalkyl(C₁₋₆); N-Alkyl(C₁₋₆)aminoalkyl(C₁₋₆)carbonylalkyl(C₁₋₆); N,N,Dialkyl(C₁₋₆)aminoalkyl(C₁₋₆)carbonylalkyl(C₁₋₈); Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; C₁₋₆-Monohydroxyalkyl; C₂₋₆-Polyhydroxyalkyl; Alkoxy(C₁₋₆)alkyl(C₁₋₆); C₁₋₆-Trifluoralkyl;
• die Gruppen R₂ und R₃, die gleich oder verschieden sein können, ein Wasserstoffatom; ein Halogenatom; eine Gruppe Z; Alkyl(C₁₋₆)carbonyl; Aminoalkyl(C₁₋₆)carbonyl; N-Z-Aminoalkyl(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; Aminoalkyl(C₁₋₆)carbonylalkyl(C₁₋₆); N-Z-Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy, Alkyl(C₁₋₆)carboxy; Alkyl(C₁₋₆)sulfonyl; Aminosulfonyl; N-Z-Aminosulfonyl; N-Alkyl(C₁₋₆)aminosulfonyl; N,N-Dialkyl(C₁₋₆)aminosulfonyl; Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆), N,N-Dialkyl(C₁₋₆)carbamoylalkyl(C₁₋₆); C₁₋₆-Alkyl; Hydroxy, Nitro, C₁₋₆-Monohydroxyalkyl; C₂₋₆-Polyhydroxyalkyl; Alkoxy(C₁₋₆)alkyl(C₁₋₆); C₁₋₆-Trifluoroalkyl; Cyano; Amino; N-Alkyl(C₁₋₆)amino; N,N-Dialkyl(C₁₋₆)amino (die beiden Alkylsubstituenten können einen 5- oder 6-gliedrigen Ring bilden); N-Hydroxyalkyl(C₁₋₆)amino; N,N-Bis(hydroxyalkyl(C₁₋₆))amino; N-Polyhydroxyalkyl(C₂₋₆)amino; N,N-Bis(polyhydroxyalkyl(C₂₋₆))amino; Aminoalkyl(C₁₋₆)amino, wobei die endständige Aminogruppe unsubstituiert oder mit einer oder zwei C₁₋₆-Alkylgruppen substituiert ist, wobei die Alkylgruppen einen gesättigten oder ungesättigten 5- oder 6-gliedrigen Ring bilden können; eine Aminogruppe, die mit Alkyl(C₁₋₆)carbonyl, Trifluoralkyl(C₁₋₆)carbonyl, Aminoalkyl(C₁₋₆)carbonyl, N-Z-Aminoalkyl(C₁₋₆)carbonyl, N-Alkyl(C₁₋₆)aminoalkyl(C₁₋₆)carbonyl, N,N-Dialkyl(C₁₋₆)aminoalkyl(C₁₋₆)carbonyl, Formyl geschützt ist, OR₄ oder SR₄;
• die Substituenten R₂ und R₃ können gemeinsam einen ungesättigten Ring der folgenden Struktur (II) bilden: 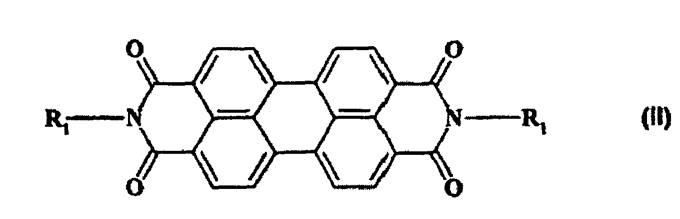
• R₄ C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Trifluoroalkyl; C₁₋₆-Aminosulfonylalkyl; N-Z-Aminosulfonylalkyl(C₁₋₆); N-Alkyl(C₁₋₆) aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); C₁₋₆-Aminoalkyl; C₁₋₆-Aminoalkyl, wobei die Aminogruppe mit einer oder zwei gleichen oder verschiedenen Gruppen substituiert ist, die unter Alkyl(C₁₋₆), Monohydroxyalkyl(C₁₋₆); Polyhydroxyalkyl(C₂₋₆), Alkyl(C₁₋₆)carbonyl, Formyl, Trifluoralkyl(C₁₋₆)-carbonyl, Alkyl(C₁₋₆)carboxy, Alkyl(C₁₋₆)sulfonyl ausgewählt sind;
• Z ist unter den ungesättigten kationischen Gruppen der folgenden Formeln (III) und (IV) und den gesättigten kationischen Gruppen der folgenden Formel (V) ausgewählt: 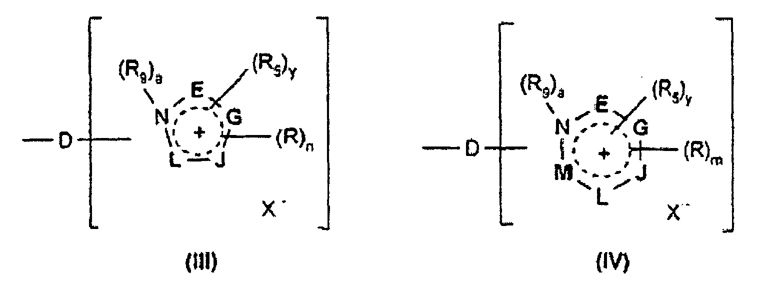 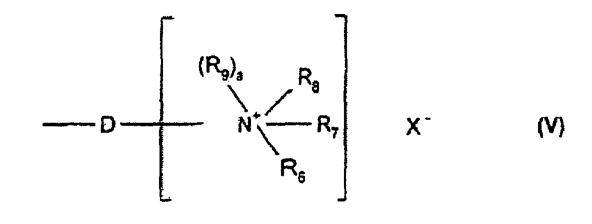 worin bedeuten:
• D eine Verbindungsgruppe, die eine geradkettige oder verzweigte Alkylgruppe mit vorzugsweise 1 bis 14 Kohlenstoffatomen ist, die durch ein oder mehrere Heteroatome, wie Sauerstoff, Schwefel oder Stickstoff, unterbrochen sein und mit einer oder mehreren Hydroxygruppen oder C₁₋₆-Alkoxygruppen substituiert sein kann und eine oder mehrere Ketofunktionen aufweisen kann;
• die Ringbestandteile E, G, J, L und M, die gleich oder verschieden sind, ein Kohlenstoffatom, Sauerstoffatom, Schwefelatom oder Stickstoffatom,
• n 0 oder eine ganze Zahl von 1 bis 4;
• m 0 oder eine ganze Zahl von 1 bis 5;
• die Gruppen R, die gleich oder verschieden sind, eine kovalente Bindung, eine Gruppe Z, ein Halogenatom, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋₆)thio, Amino, eine Aminogruppe, die mit Alkyl(C₁₋₆)carbonyl, Carbamoyl oder Alkyl(C₁₋₆)sulfonyl geschützt ist, eine Gruppe NHR" oder NR''R''', worin die Gruppen R" und R''', die gleich oder verschieden sind, C₁₋₆-Alkyl, C₁₋₆-Monohydroxyalkyl oder C₂₋₆-Polyhydroxyalkyl bedeuten;
wenn in den Formeln (III) oder (IV) n oder m größer als 2 ist, können zwei benachbarte Gruppen R auch gemeinsam einen ungesättigten, 5- oder 6-gliedrigen Ring auf Kohlenstoffbasis oder Ring, der ein oder mehrere Heteroatome enthält, bilden;
• R₅ eine kovalente Bindung, C₁₋₆-Alkyl, C₁₋₆-Monohydroxyalkyl, C₂₋₆-Polyhydroxyalkyl, C₁₋₆-Cyanoalkyl, Trialkyl(C₁₋₆)silanalkyl(C₁₋₆), C₁₋₆-Alkoxy(C₁₋₆)alkyl(C₁₋₆), Carbamoylalkyl(C₁₋₆), Alkyl(C₁₋₆)carboxyalkyl(C₁₋₆), Benzyl oder eine Gruppe Z;
• die Gruppen R₆, R₇ und R₈, die gleich oder verschieden sind, eine kovalente Bindung, C₁₋₆-Alkyl, C₁₋₆-Monohydroxyalkyl; C₂₋₆-Polyhydroxyalkyl; Alkoxy(C₁₋₆)alkyl(C₁₋₆); C₁₋₆-Cyanoalkyl; Aryl, Benzyl, C₁₋₆-Amidoalkyl, Trialkyl(C₁₋₆)silanalkyl(C₁₋₆), C₁₋₆-Aminoalkyl, wobei die Aminogruppe mit Alkyl(C₁₋₆)carbonyl, Carbamoyl oder Alkyl(C₁₋₆)sulfonyl geschützt ist; wobei zwei der Gruppen R₆, R₇ und R₈ auch gemeinsam mit dem Stickstoffatom, an das sie gebunden sind, einen gesättigten, 5- oder 6-gliedrigen Ring auf Kohlenstoffbasis oder Ring, der ein oder mehrere Heteroatome enthält, bilden können;
wobei eine der Gruppen R₆, R₇ und R₈ auch eine zweite Gruppe Z bedeuten kann, die von der ersten Gruppe Z verschieden oder mit ihr identisch ist;
• R₉ C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoroalkyl; Trialkyl(C₁₋₆)silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
• a und y 0 oder 1, mit den folgenden Maßgaben:
- in den ungesättigten kationischen Gruppen der Formel (III):
- wenn a = 0, ist die Verbindungsgruppe D an das Stickstoffatom gebunden;
- wenn a = 1, ist die Verbindungsgruppe D an einen Ringbestandteil E, G, J oder L gebunden,
- y kann nur unter den folgenden Bedingungen 1 bedeuten:
1) wenn die Ringbestandteile E, G, J und L ein gleichzeitig ein Kohlenstoffatom bedeuten und die Gruppe R₅ an dem Stickstoffatom des ungesättigten Rings gebunden ist; oder
2) wenn mindestens ein Ringbestandteil E, G, J und L Stickstoffatom bedeutet, an dem die Gruppe R₅ gebunden ist;
- in den ungesättigten kationischen Gruppen der Formel (IV):
- wenn a = 0, ist die Verbindungsgruppe D an das Stickstoffatom gebunden,
- wenn a = 1, ist die Verbindungsgruppe D an einen der Ringbestandteile E, G, J, L oder M gebunden,
- y kann nur den Wert 1 annehmen, wenn mindestens ein Ringbestandteil E, G, J, L oder M ein zweiwertiges Atom bedeutet und die Gruppe Rs an dem Stickstoffatom des ungesättigten Rings gebunden ist;
- in den kationischen Gruppen der Formel (V):
- wenn a = 0, ist die Verbindungsgruppe D an das Stickstoffatom gebunden, das die Gruppen R₇ bis R₈ trägt,
- wenn a = 1, bilden zwei der Gruppen R₇ bis R₈ gemeinsam mit dem Stickstoffatom, an das sie gebunden sind, einen gesättigten Ring mit 5 oder 6 Ringbestandteilen, wie er oben definiert wurde, und die Verbindungsgruppe D befindet sich an einem Kohlenstoffatom des ungesättigten Rings;
• X⁻ ein einwertiges oder zweiwertiges Anion;
mit der Maßgabe, dass:
- die Anzahl der kationischen Gruppen Z in der Formel (III), (IV) oder (V) mindestens 1 beträgt.

2. Verwendung nach Anspruch 1, **dadurch gekennzeichnet, dass** es sich bei den Keratinsubstanzen um die menschliche Haut, die Lippen und die Nägel handelt.

3. Verwendung nach Anspruch 1, **dadurch gekennzeichnet, dass** die Keratinsubstanzen menschliche Keratinfasern sind, beispielsweise Haare, Körperhaare, Wimpern und Augenbrauen.

4. Verwendung nach einem der Ansprüche 1 bis 3, **dadurch gekennzeichnet, dass** die Ringe der ungesättigten Gruppen Z der Formel (III) unter Pyrrol, Imidazol, Pyrazol, Oxazol, Thiazol und Triazol ausgewählt sind.

5. Verwendung nach einem der Ansprüche 1 bis 3, **dadurch gekennzeichnet, dass** die Ringe der ungesättigten Gruppen Z der Formel (IV) unter Pyridin, Pyrimidin, Pyrazin, Oxazin und Triazin ausgewählt sind.

6. Verwendung nach einem der vorhergehenden Ansprüche, **dadurch gekennzeichnet, dass** in der Formel (V) zwei der Gruppen R₆, R₇ und R₈ einen Pyrrolidinring, Piperidinring, Piperazinring oder Morpholinring bilden, wobei der Ring unsubstituiert vorliegen kann oder mit einem Halogenatom, einer Hydroxygruppe, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Ketoalkyl, Thio, C₁₋₆-Thioalkyl, Alkyl(C₁₋₆)thio, Amino und Aminogruppen, die A mit Alkyl(C₁₋₆) carbonyl, Carbamoyl oder Alkyl(C₁₋₆)sulfonyl geschützt sein kann, substituiert sein können.

7. Verwendung nach einem der vorhergehenden Ansprüche, **dadurch gekennzeichnet, dass** X⁻ unter den Halogenen, Hydroxid, Hydrogensulfat oder Alkyl(C₁₋₆)sulfaten ausgewählt ist.

8. Verwendung nach einem der vorhergehenden Ansprüche, **dadurch gekennzeichnet, dass** in der Formel (I) die Gruppen R₂ und R₃ gemeinsam einen ungesättigten Ring der folgenden Formel (II) bilden 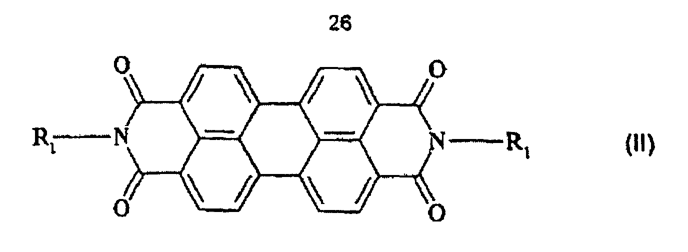 worin die Gruppe Ri die oben für die Formel (I) angegebenen Bedeutungen aufweist.

9. Verwendung nach einem der vorhergehenden Ansprüche, **dadurch gekennzeichnet, dass** die Verbindungen der Formel (I) unter den folgenden Verbindungen ausgewählt sind:
N,N'-Dihexyl-1,3,8,10-tetrahydro-N,N,N',N'-tetramethyl-1,3,8,10-tetraoxo-anthra[2,1,9-def:6,5,10-d'e'f']diisochinolin-2,9-dipropanaminiumdiiodid;
4,4'-[(1,3,8,10-Tetrahydro-1,3,8,10-tetraoxoanthra[2,1,9-def:6,5,10,-d'e'f']diisochinolin-2,9-diyl)di-3,1-propandiyl]bis[4-methyl]morpholinium-diiodid;
N,N,N',N'-Tetraethyl-1,3,8,10-tetrahydro-N,N'-dimethyl-1,3,8,10-tetraoxo-anthra[2,1,9-def:6,5,10-d'e'f']diisochinolin-2,9-dipropanaminium-diiodid;
N,N,N,N',N',N'-Hexaethyl-1,3,8,10-tetrahydro-1,3,8,10-tetraoxoanthra[2,1,9-def:6,5,10-d'e'f']diisochinolin-2,9-diethanaminiumdiiodid;
N,N'-Diethyl-1,3,8,10-tetrahydro-N,N,N',N'-tetramethyl-1,3,8,10-tetraoxo-anthra[2,1,9-def:6,5,10-d'e'f']diisochinolin-2,9-dipropanaminium-diiodid;
1,3,8,10-Tetrahydro-N,N,N,N',N',N'-hexamethyl-1,3,8,10-tetraoxo-anthra[2,1,9-def:6,5,10-d'e'f']diisochinolin-2,9-diethanaminium-diiodid;
1,3,8,10-Tetrahydro-N,N,N,N',N',N'-hexamethyl-1,3,8,10-tetraoxo-anthra[2,1,9-def:6,5,10-d'e'f']diisochinolin-2,9-dipropanaminium-diiodid;
N,N,N,N',N',N'-Hexaethyl-1,3,8,10-tetrahydro-1,3,8,10-tetraoxoanthra[2,1,9-def:6,5,10-d'e'f']diisochinolin-2,9-diethanaminiumdibenzolsulfonat;
1,3,8,10-Tetrahydro-N,N,N,N',N',N'-hexaethyl-1,3,8,10-tetraoxoanthra[2,1,9-def:6,5,10-d'e'f']diisochinolin-2,9-diethanaminiumdiiodid;
1,3,8,10-Tetrahydro-N,N,N,N',N',N'-hexamethyl-1,3,8,10-tetraoxo-anthra[2,1,9-def:6,5,10-d'e'f']diisochinolin-2,9-diethanaminium-dibenzolsulfonat;
1,3,8,10-Tetrahydro-N,N,N,N',N',N'-hexamethyl-1,3,8,10-tetraoxo-anthra[2,1,9-def:6,5,10-d'e'f']diisochinolin-2,9-diethanaminium-diiodid;
Bis[1,1'-methyl]-1,1'-[(1,3,8,10-tetrahydro-1,3,8,10-tetraoxoanthra[2,1,9-def:6,5,10-d'e'f']diisochinolin-2,9-diyl)di-3,1-propandiyl]-imidazolinium-diiodid;
Bis[ 1,1'-methyl]-1,1'-[(1,3,8,10-tetrahydro-1,3,8, 10-tetraoxoanthra[2,1,9-def:6,5,10-d'e'f']diisochinolin-2,9-diyl)di-3,1-propandiyl]-imidazolinium-dimethosulfat.

10. Verwendung nach Anspruch 9, **dadurch gekennzeichnet, dass** die Verbindungen der Formel (I) unter den folgenden Verbindungen ausgewählt sind:
1,3,8,10-Tetrahydro-N,N,N,N',N',N'-hexamethyl-1,3,8,10-tetraoxo-anthra[2,1,9-def:6,5,10-d'e'f']diisochinolin-2,9-diethanaminium-diiodid;
Bis[1,1'-methyl]-1,1'-[(1,3,8,10-tetrahydro-1,3,8,10-tetraoxoanthra[2,1,9-def:6,5,10-d'e'f]diisochinolin-2,9-diyl)di-3,1-propandiyl]-imidazolinium-diiodid;
Bis[1,1'-methyl]-1,1'-[(1,3,8,10-tetrahydro-1,3,8,10-tetraoxoanthra[2,1,9-def:6,5,10-d'e'f']diisochinolin-2,9-diyl)di-3,1-propandiyl]-imidazolinium-dimethosulfat.

11. Verwendung nach einem der vorhergehenden Ansprüche, **dadurch gekennzeichnet, dass** die Additionssalze der Verbindungen der Formel (I) mit einer Säure unter den Hydrochloriden, Hydrobromiden, Sulfaten, Citraten, Succinaten, Tartraten, Lactaten und Acetaten ausgewählt sind.

12. Zusammensetzung zum Färben oder Schminken von Keratinsubstanzen, **dadurch gekennzeichnet, dass** sie in einem zum Färben oder Schminken geeigneten Medium mindestens eine Verbindung der Formel (I) nach einem der Ansprüche 1 bis 11 in einer wirksamen Menge enthält.

13. Zusammensetzung zum direkten Färben von menschlichen Keratinfasern und insbesondere zum Färben der Haare, **dadurch gekennzeichnet, dass** sie in einem zum Färben geeigneten Medium mindestens eine Verbindung der Formel (I) nach einem der Ansprüche 1 bis 11 in einer wirksamen Menge enthält.

14. Zusammensetzung nach Anspruch 13, **dadurch gekennzeichnet, dass** sie einen pH-Wert von 3 bis 12 aufweist.

15. Zusammensetzung zum Färben nach einem der Ansprüche 12 bis 14, **dadurch gekennzeichnet, dass** die Verbindungen der Formel (I) in einer Konzentration von 0,005 bis 12 Gew.-%, bezogen auf das Gesamtgewicht der Zusammensetzung, enthalten sind.

16. Zusammensetzung nach Anspruch 15, **dadurch gekennzeichnet, dass** die Verbindungen der Formel (I) in einer Konzentration von 0,05 bis 6 Gew.-%, bezogen auf das Gesamtgewicht der Zusammensetzung, vorliegen.

17. Zusammensetzung nach einem der Ansprüche 13 bis 16, **dadurch gekennzeichnet, dass** das zum Färben geeignete Medium ein wässeriges Medium ist, das aus Wasser und/oder organischen Lösungsmitteln in Mengenanteilen von 1 bis 40 Gew.-%, bezogen auf das Gesamtgewicht der Zusammensetzung, besteht.

18. Zusammensetzung zum Schminken nach Anspruch 12, **dadurch gekennzeichnet, dass** die Verbindungen der Formel (I) in einer Konzentration von 0,005 bis 25 Gew.-%, bezogen auf das Gesamtgewicht der Zusammensetzung, enthalten sind.

19. Zusammensetzung nach Anspruch 12 oder 18, **dadurch gekennzeichnet, dass** sie als Produkt zum Schminken der menschlichen Haut, der Lippen, der Nägel, der Wimpern oder der Augenbrauen vorliegt.

20. Zusammensetzung nach Anspruch 19, **dadurch gekennzeichnet, dass** sie als Make-up, Wangenrouge, Lidschatten, Puder, Blush, Lippenstift oder Lipgloss, Nagellack, Mascara oder Eyeliner vorliegt.

21. Verfahren zum Färben von Keratinfasern und insbesondere menschlichen Keratinfasern, wie Haaren, durch direkte Färbung, **dadurch gekennzeichnet, dass** auf die trockenen oder feuchten Keratinfasern eine Farbmittelzusammensetzung nach einem der Ansprüche 13 bis 17 aufgetragen wird und die Fasern ohne zwischenzeitliches Spülen getrocknet werden.

22. Verfahren zum Färben von Keratinfasern und insbesondere menschlichen Keratinfasern, insbesondere Haaren durch direkte Färbung, **dadurch gekennzeichnet, dass** auf die trockenen oder feuchten Keratinfasern eine Farbmittelzusammensetzung nach einem der Ansprüche 13 bis 17 aufgetragen wird und, nachdem die Zusammensetzung gegebenenfalls während einer Einwirkzeit von 3 bis 30 min auf die Fasern einwirken gelassen wurde, die Fasern gespült, gegebenenfalls gewaschen, nochmals gespült und dann getrocknet werden.

## Claims

1. Use, as direct dyes in, or for the manufacture of, dye compositions for keratin substances, of fused polycyclic compounds of formula (I) below, and the addition salts thereof with an acid: 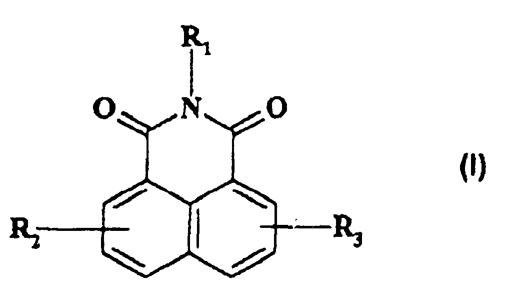 in which:
• R₁ represents a hydrogen atom; a group Z defined below; an amino(C₁-C₆)alkylcarbonyl(C₁-C₆)alkyl radical; an N-Z-amino(C₁-C₆)alkylcarbonyl(C₁-C₆)alkyl radical; an N-(C₁-C₆)alkylamino(C₁-C₆)alkylcarbonyl(C₁-C₆)alkyl radical; an N,N-di(C₁-C₆)alkylamino(C₁-C₆)alkylcarbonyl(C₁-C₆)alkyl radical; an aminosulphonyl(C₁-C₆)alkyl radical; an N-Z-aminosulphonyl(C₁-C₆)alkyl radical; an N-(C₁-C₆)alkylaminosulphonyl(C₁-C₆)alkyl radical; an N,N-di(C₁-C₆)alkylaminosulphonyl(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical;
• R₂ and R₃, which may be identical or different, represent a hydrogen atom; a halogen atom; a group Z; a (C₁-C₆)alkylcarbonyl radical; an amino(C₁-C₆)alkylcarbonyl radical; an N-Z-amino(C₁-C₆)alkylcarbonyl radical; an N-(C₁-C₆)alkylamino(C₁-C₆)alkylcarbonyl radical; an N,N-di(C₁-C₆)alkylamino(C₁-C₆)alkylcarbonyl radical; an amino(C₁-C₆)alkylcarbonyl(C₁-C₆)alkyl radical; an N-Z-amino(C₁-C₆)alkylcarbonyl(C₁-C₆)alkyl radical; an N-(C₁-C₆)alkylamino(C₁-C₆)alkylcarbonyl(C₁-C₆)alkyl radical; an N,N-di(C₁-C₆)alkylamino(C₁-C₆)alkylcarbonyl(C₁-C₆)alkyl radical; a carboxyl radical; a (C₁-C₆)alkylcarboxy radical; a C₁-C₆ alkylsulphonyl radical; an aminosulphonyl radical; an N-Z-aminosulphonyl radical; an N-(C₁-C₆)alkylaminosulphonyl radical; an N,N-di(C₁-C₆)alkylaminosulphonyl radical; an aminosulphonyl (C₁-C₆)alkyl radical; an N-Z-aminosulphonyl(C₁-C₆)alkyl radical; an N-(C₁-C₆)alkylaminosulphonyl(C₁-C₆)alkyl radical; an N,N-di(C₁-C₆)alkylaminosulphonyl(C₁-C₆)alkyl radical; a carbamyl radical; an N-(C₁-C₆)alkylcarbamyl radical; an N,N-di(C₁-C₆)alkylcarbamyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ alkyl radical; a hydroxyl radical; a nitro radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; an amino radical; an N-(C₁-C₆)alkylamino radical; an N,N-di(C₁-C₆)alkylamino radical (the two alkyl substituents possibly forming a 5- or 6-membered ring); an N-hydroxy(C₁-C₆)alkylamino radical; an N,N-bis(hydroxy(C₁-C₆)alkyl)amino radical; an N-polyhydroxy(C₂-C₆)alkylamino radical; an N,N-bis(polyhydroxy(C₂-C₆)alkyl) amino radical; an amino (C₁-C₆)alkylamino radical in which the terminal amino group is unsubstituted or substituted with one or two C₁-C₆ alkyl radicals, the said alkyl radicals possibly forming a saturated or unsaturated 5- or 6-membered ring; an amino group protected with a (C₁-C₆)-alkylcarbonyl, trifluoro(C₁-C₆)alkylcarbonyl, amino(C₁-C₆)alkylcarbonyl, N-Z-amino(C₁-C₆)alkyl-carbonyl, N-(C₁-C₆)alkylamino(C₁-C₆)alkylcarbonyl, N,N-di(C₁-C₆)-alkylamino(C₁-C₆)alkylcarbonyl or formyl radical; a group OR₄ or SR₄;
• the substituents R₂ and R₃ may together form an unsaturated ring of structure (II) below: 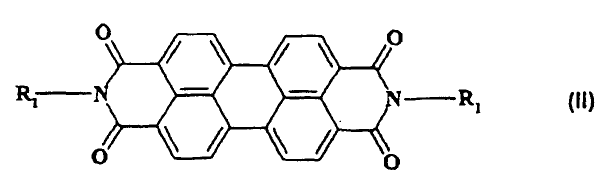
• R₄ denotes a C₁-C₆ alkyl radical; a C₁-C₆ monohydroxyalkyl radical; a C₂-C₆ polyhydroxyalkyl radical; a group Z; a (C₁-C₆)alkoxy(C₁-C₆) alkyl radical; an aryl radical; a benzyl radical; a carboxy(C₁-C₆)alkyl radical; a (C₁-C₆)alkylcarboxy(C₁-C₆)alkyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ trifluoroalkyl radical; a C₁-C₆ aminosulphonylalkyl radical; a C₁-C₆ N-Z-aminosulphonylalkyl radical; an N-(C₁-C₆)alkylaminosulphonyl(C₁-C₆)alkyl radical; an N,N-di(C₁-C₆)alkylaminosulphonyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro(C₁-C₆)alkylcarbonyl, (C₁-C₆)alkylcarboxyl, and C₁-C₆ alkylsulphonyl radicals;
• Z is chosen from the unsaturated cationic groups of formulae (III) and (IV) below, and the saturated cationic groups of formula (V) below: 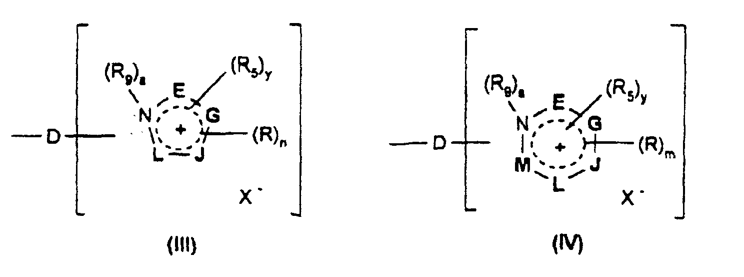 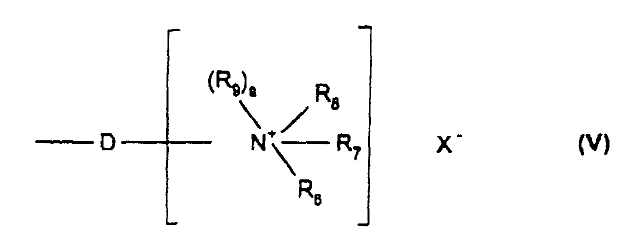 in which:
• D is a linker arm which represents a linear or branched alkyl chain preferably containing from 1 to 14 carbon atoms, which can be interrupted by one or more hetero atoms such as oxygen, sulphur or nitrogen atoms, and which can be substituted with one or more hydroxyl or C₁-C₆ alkoxy radicals, and which can bear one or more ketone functions;
• the ring members E, G, J, L and M, which may be identical or different, represent a carbon, oxygen, sulphur or nitrogen atom;
• n is an integer between 0 and 4 inclusive;
• m is an integer between 0 and 5 inclusive;
• the radicals R, which may be identical or different, represent a covalent bond, a group Z, a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehydo radical, a carboxyl radical, a (C₁-C₆)alkylcarbonyl radical, a thio radical, a C₁-C₆ thioalkyl radical, a C₁-C₆ alkylthio radical, an amino radical, an amino radical protected with a (C₁-C₆)alkylcarbonyl, carbamyl or C₁-C₆ alkylsulphonyl radical; a group NHR" or NR''R''' in which R" and R"', which may be identical or different, represent a C₁-C₆ alkyl radical, a C₁-C₆ monohydroxyalkyl radical or a C₂-C₆ polyhydroxyalkyl radical; when, in formula (III) or (IV), n or m is greater than 2, two adjacent radicals R can together also form an unsaturated 5- or 6-membered ring which is carbon-based or which contains one or more hetero atoms;
• R₅ represents a covalent bond, a C₁-C₆ alkyl radical, a C₁-C₆ monohydroxyalkyl radical, a C₂-C₆ polyhydroxyalkyl radical, a cyano(C₁-C₆)alkyl radical, a tri(C₁-C₆)alkylsilane(C₁-C₆)alkyl radical, a (C₁-C₆)alkoxy(C₁-C₆)alkyl radical, a carbamyl (C₁-C₆) alkyl radical, a (C₁-C₆)alkylcarboxy(C₁-C₆)alkyl radical, a benzyl radical or a group Z;
• R₆, R₇ and R₈, which may be identical or different, represent a covalent bond, a C₁-C₆ alkyl radical, a C₁-C₆ monohydroxyalkyl radical, a C₂-C₆ polyhydroxyalkyl radical, a (C₁-C₆)alkoxy(C₁-C₆)alkyl radical, a cyano(C₁-C₆)alkyl radical, an aryl radical, a benzyl radical, a C₁-C₆ amidoalkyl radical, a tri(C₁-C₆)alkylsilane(C₁-C₆)alkyl radical or a C₁-C₆ aminoalkyl radical in which the amine is protected with a (C₁-C₆)alkylcarbonyl, carbamyl or C₁-C₆ alkylsulphonyl radical; two of the radicals R₆, R₇ and R₈ can together also form, with the nitrogen atom to which they are attached, a saturated 5- or 6-membered ring which is carbon-based or contains one or more hetero atoms, one of the radicals R₆, R₇ and R₈ can also represent a second group Z which is identical to or different from the first group Z;
• R₉ represents a C₁-C₆ alkyl radical; a C₁-C₆ monohydroxy-alkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy(C₁-C₆)alkyl radical; a cyano(C₁-C₆)alkyl radical; a carbamyl(C₁-C₆)alkyl radical; a C₁-C₆ trifluoroalkyl radical; a tri(C₁-C₆)alkylsilane-(C₁-C₆)alkyl radical; a C₁-C₆ sulphonamidoalkyl radical; a (C₁-C₆)alkylcarboxy(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylketo(C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• a and y are integers equal to 0 or 1; with the following conditions:
- in the unsaturated cationic groups of formula (III):
- when a = 0, the linker arm D is attached to the nitrogen atom,
- when a = 1, the linker arm D is attached to one of the ring members E, G, J or L,
- y can take the value 1 only:
1) when the ring members E, G, J and L simultaneously represent a carbon atom and when the radical R₅ is borne by the nitrogen atom of the unsaturated ring; or alternatively
2) when at least one of the ring members E, G, J and L represents a nitrogen atom to which the radical R₅ is attached;
- in the unsaturated cationic groups of formula (IV) :
- when a = 0, the linker arm D is attached to the nitrogen atom,
- when a = 1, the linker arm D is attached to one of the ring members E, G, J, L or M,
- y can take the value 1 only when at least one of the ring members E, G, J, L and M represents a divalent atom and when the radical R₅ is borne by the nitrogen atom of the unsaturated ring;
- in the cationic groups of formula (V) :
- when a = 0, then the linker arm D is attached to the nitrogen atom bearing the radicals R₇ and R₈,
- when a = 1, then two of the radicals R₇ and R₈ form, together with the nitrogen atom to which they are attached, a saturated 5- or 6-membered ring as defined above, and the linker arm D is borne by a carbon atom of the said saturated ring;
• X⁻ represents a monovalent or divalent anion;
**it being understood that:**
- the number of cationic groups Z of formula (III), (IV) or (V) is at least equal to 1.

2. Use according to Claim 1, **characterized in that** the keratin substances denote human skin, the lips and the nails.

3. Use according to Claim 1, **characterized in that** the keratin substances denote human keratin fibres such as head hair, body hair, the eyelashes and the eyebrows.

4. Use according to any one of Claims 1 to 3, **characterized in that** the rings of the unsaturated groups Z of formula (III) are chosen from pyrrole, imidazole, pyrazole, oxazole, thiazole and triazole rings.

5. Use according to any one of Claims 1 to 3, **characterized in that** the rings of the unsaturated groups Z of formula (IV) are chosen from pyridine, pyrimidine, pyrazine, oxazine and triazine rings.

6. Use according to any one of the preceding claims, **characterized in that**, in formula (V), two of the radicals R₆, R₇ and R₈ form a pyrrolidine ring, a piperidine ring, a piperazine ring or a morpholine ring, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehydo radical, a carboxyl radical, a keto(C₁-C₆)alkyl radical, a thio radical, a C₁-C₆ thioalkyl radical, a C₁-C₆ alkylthio radical, an amino radical or an amino radical protected with a (C₁-C₆)alkylcarbonyl, carbamyl or C₁-C₆ alkylsulphonyl radical.

7. Use according to any one of the preceding claims, **characterized in that** X⁻ is chosen from a halogen atom, a hydroxide, a hydrogen sulphate or a (C₁-C₆)alkyl sulphate.

8. Use according to any one of the preceding claims, **characterized in that**, in formula (I), R₂ and R₃ together form an unsaturated ring of formula (II) below: 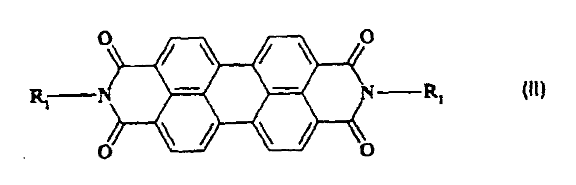 in which the radical R₁ has the same meanings as in formula (I).

9. Use according to any one of the preceding claims, **characterized in that** the compounds of formula (I) are chosen from the following:
N,N'-dihexyl-1,3,8,10-tetrahydro-N,N,N',N'-tetramethyl-1,3,8,10-tetraoxoanthra[2,1,9-def:6,5,10-d'e'f']diisoquinoline-2,9-dipropanaminium diiodide;
4,4'-[(1,3,8,10-tetrahydro-1,3,8,10-tetraoxoanthra[2,1,9-def:6,5,10-d'e'f]diisoquinoline-2,9-diyl)di-3,1-propanediyl]bis[4-methyl]morpholinium diiodide;
N,N,N',N'-tetraethyl-1,3,8,10-tetrahydro-N,N'-dimethyl-1,3,8,10-tetraoxoanthra[2,1,9-def:6,5,10-d'e'f']diisoquinoline-2,9-dipropanaminium diiodide;
N,N,N,N',N',N'-hexaethyl-1,3,8,10-tetrahydro-1,3,8,10-tetraoxoanthra[2,1,9-def:6,5,10-d'e'f']diisoquinoline-2,9-diethanaminium diiodide;
N,N'-diethyl-1,3,8,10-tetrahydro-N,N,N',N'-tetramethyl-1,3,8,10-tetraoxoanthra[2,1,9-def:6,5,10-d'e'f']diisoquinoline-2,9-dipropanaminium diiodide;
1,3,8,10-tetrahydro-N,N,N,N',N',N'-hexamethyl-1,3,8,10-tetraoxoanthra[2,1,9-def:6,5,10-d'e'f']diisoquinoline-2,9-diethanaminium diiodide;
1,3,8,10-tetrahydro-N,N,N,N',N',N'-hexamethyl-1,3,8,10-tetraoxoanthra[2,1,9-def:6,5,10-d'e'f']diisoquinoline-2,9-dipropanaminium diiodide;
N,N,N,N',N',N'-hexaethyl-1,3,8,10-tetrahydro-1,3,8,10-tetraoxoanthra[2,1,9-def:6,5,10-d'e'f']diisoquinoline-2,9-diethanaminium dibenzenesulphonate;
1,3,8,10-tetrahydro-N,N,N,N',N',N'-hexaethyl-1,3,8,10-tetraoxoanthra[2,1,9-def:6,5,10-d'e'f']diisoquinoline-2,9-diethanaminium diiodide;
1,3,8,10-tetrahydro-N,N,N,N',N',N'-hexamethyl-1,3,8,10-tetraoxoanthra[2,1,9-def:6,5,10-d'e'f']diisoquinoline-2,9-diethanaminium dibenzenesulphonate;
1,3,8,10-tetrahydro-N,N,N,N',N',N'-hexamethyl-1,3,8,10-tetraoxoanthra[2,1,9-def:6,5,10-d'e'f']diisoquinoline-2,9-diethanaminium diiodide;
bis[1-1'-methyl]-1,1'-[(1,3,8,10-tetrahydro-1,3,8,10-tetraoxoanthra[2,1,9-def:6,5,10-d'e'f]diisoquinoline-2,9-diyl)di-3,1-propanediyl]imidazolinium diiodide;
bis[1-1'-methyl]-1,1'-[(1,3,8,10-tetrahydro-1,3,8,10-tetraoxoanthra[2,1,9-def:6,5,10-d'e'f]diisoquinoline-2,9-diyl)di-3,1-propanediyl]imidazolinium dimethosulphate.

10. Use according to Claim 9, **characterized in that** the compounds of formula (I) are chosen from the following:
1,3,8,10-tetrahydro-N,N,N,N',N',N'-hexamethyl-1,3,8,10-tetraoxoanthra[2,1,9-def:6,5,10-d'e'f']diisoquinoline-2,9-diethanaminium diiodide;
bis[1-1'-methyl]-1,1'-[(1,3,8,10-tetrahydro-1,3,8,10-tetraoxoanthra[2,1,9-def:6,5,10-d'e'f]diisoquinoline-2,9-diyl)di-3,1-propanediyl]imidazolinium diiodide;
bis[1-1'-methyl]-1,1'-[(1,3,8,10-tetrahydro-1,3,8,10-tetraoxoanthra[2,1,9-def:6,5,10-d'e'f]diisoquinoline-2,9-diyl)di-3,1-propanediyl]imidazolinium dimethosulphate.

11. Use according to any one of the preceding claims, **characterized in that** the addition salts with an acid of the compounds of formula (I) are chosen from the hydrochlorides, hydrobromides, sulphates, citrates, succinates, tartrates, lactates and acetates.

12. Composition for dyeing or making up keratin substances, **characterized in that** it comprises, in a medium which is suitable for dyeing or for make-up, an effective amount of at least one compound of formula (I) defined in any one of Claims 1 to 11.

13. Direct dye composition for human keratin fibres and in particular the hair, **characterized in that** it comprises, in a medium which is suitable for dyeing, an effective amount of at least one compound of formula (I) defined in any one of Claims 1-11.

14. Composition according to Claim 13, **characterized in that** it has a pH of between 3 and 12.

15. Dye composition according to any one of Claims 12 to 14, **characterized in that** the compounds of formula (I) are present in a concentration ranging from 0.005 to 12% by weight relative to the total weight of the composition.

16. Composition according to Claim 15, **characterized in that** the compounds of formula (I) are present in a concentration ranging from 0.05 to 6% by weight relative to the total weight of the composition.

17. Composition according to any one of Claims 13 to 16, **characterized in that** the medium which is suitable for dyeing is an aqueous medium consisting of water and/or of organic solvents, in proportions of between 1 and 40% by weight relative to the total weight of the composition.

18. Make-up composition according to Claim 12, **characterized in that** the compounds of formula (I) are present in a concentration ranging from 0.005 to 25% by weight relative to the total weight of the composition.

19. Composition according to Claim 12 or 18, **characterized in that** it is in the form of a make-up product for human skin, the lips, the nails, the eyelashes or the eyebrows.

20. Composition according to Claim 19, **characterized in that** it is in the form of a foundation, a face powder, an eyeshadow, a rouge, a blusher, a lipstick, a lip gloss, a nail varnish, a mascara or an eyeliner.

21. Process for dyeing keratin fibres, and in particular human keratin fibres and especially the hair, by direct dyeing, **characterized in that** a dye composition as defined in any one of Claims 13 to 17 is applied to the wet or dry keratin fibres, and these fibres are dried without intermediate rinsing.

22. Process for dyeing keratin fibres, and in particular human keratin fibres and especially the hair, by direct dyeing, **characterized in that** a dye composition as defined in any one of Claims 13 to 17 is applied to the wet or dry keratin fibres and, after the composition has optionally been left on the fibres for an exposure time ranging from 3 to 60 minutes, the fibres are rinsed, optionally washed, and then rinsed again and dried.
